# EUROPEAN PATENT SPECIFICATION

(11) **EP 1 369 121 B1**
(45) Date of publication and mention of the grant of the patent: **24.01.2007**
(21) Application number: 03010888.0
(22) Date of filing: 15.05.2003
(51) Int. Cl.: A61K 33/06, A61K 33/00, A61K 45/06

(54) **Use of zeolites for the preparation of oral compositions for the treatment of poisoning**
Verwendung von Zeoliten zur Herstellung oraler Zubereitungen zur Behandlung von Vergiftung
Utilisation des zeolites pour la preparation des compositions pour le traitement de l'intoxication

(30) Priority: 07.06.2002 IT MI20021246
(43) Date of publication of application: 10.12.2003
(73) Proprietor: Geomedical S.r.l., 01010 Marta, (Viterbo) (IT)
(72) Inventor: Fanelli, Mauro, 01010 Marta (VT) (IT)
(74) Representative: Minoja, Fabrizio

(56) References cited:
- EP-A- 0 759 290
- US-A- 5 264 225

## Description

This invention relates to the use of zeolites for the preparation of pharmaceutical compositions for the treatment of poisoning.

Zeolites belong to the family of tektosilicates; specifically, they are aluminosilicate hydrates of alkaline or alkaline earth metals.

Zeolites have a variety of industrial applications due to their ability to adsorb small molecules into their cavities and the fact that they can be used as molecular sieves with ion-exchange capacity and as a support in industrial catalysis.

Recently, groups of Russian researchers also described possible therapeutic uses of particular zeolites, such as clinoptilolite and heulandite (RU 2114625, US 6287579 and RU 2115421), and their immunostimulating and lipid-reducing properties and biostimulating properties in general. According to these researchers, the beneficial properties of zeolites, which can be administered alone or with nutrients of plant origin, are attributable to their ability to supply micro- and macro-elements to the body and their absorption and ion exchange capacity, which restores the normal mineral composition of the body. The zeolites used for this purpose are subjected to extreme micronisation which alters their crystalline component, and then treated with mineral fractions of silver, zinc and manganese salts with organic fractions (vitamin B12, plant extracts, etc.) in order to enhance their application efficacy. The zeolites considered in the prior art have a Si/Al ratio of between 2.5 and 5, and must therefore be considered acid. Their cation exchange capacity (CEC) is consequently low (under 200 meq/100 g).

EP 759290 discloses topical gels of minerals of volcanic origin for cosmetic and dermatological use.

US 5264225 discloses the use of zeolites combined with diatomaceous earth for adsorbing radioactive or toxic metals.

This invention relates to the use of a zeolite found in the vicinity of Lake Bolsena (Italy), having the following composition: 60% chabazite + phillipsite, 18% sanidine, 15% biotite and 7% analcime.

Phillipsite is a zeolite commonly found in Italy: it takes the form of tabular or columnar crystals and is a calcium aluminosilicate, unlike chabazite, which is a sodium aluminosilicate, consisting of rhombohedral crystals very similar to cubes, and consequently known as cubic zeolite. Analcime, which is widely considered to be a feldspathoid and not strictly a zeolite, takes the form of cubic crystals with icositetrahedral faces, consisting of sodium and potassium aluminosilicates.

The ore containing the zeolites specified above is subjected to a treatment that enhances their essential catalytic, detoxifying and ion-exchange properties for the purposes of the proposed use. In particular, the treatment according to the invention involves the following steps:
a. Suspension of zeolite ore in mineral water with high electrical conductivity at pH 7.25, or alternatively in sulphate-calcium-magnesium acid mineral water at pH 2.85;
b. Stirring of the suspension for 12-36 hours at 30-40°C;
c. Cold micronisation;
d. Drying of the micronised zeolite ore at 30-36°C to a residual water content below 10% by weight.

"Mineral water with high electrical conductivity" preferably means bicarbonate-sulphate-alkaline-magnesium water with conductivity of up to 3000 Sm⁻¹.

On the other hand, the conductivity of acid mineral water is typically below approx. 1800 Sm⁻¹. Mineral waters of volcanic origin totally unadulterated by artificial means will be used in each case.

Micronisation can advantageously be performed by the hydrocryocrushing technique followed by various sequential vibration screening steps to remove the unsuitable solid constituents. The powders thus obtained can also be subjected to the action of intense magnetic fields, then dried at temperatures below 36°C for one or two weeks to a water content below 10%, and finally packaged, preferably under vacuum. All the operations described are strictly conducted in such a way as not to alter the original composition of the natural mineral in any way, and therefore without causing the slightest artificial alteration to it.

The micronised zeolites thus obtained have a cation exchange capacity exceeding 100 meq/100g, more particularly greater than 200 meq/100 g and up to 700 meq/mg, and a very high redox potential of approx. -160 mV.

These properties contribute to give the zeolites of the invention their beneficial and therapeutic activities, especially detoxifying activity.

The zeolites of the invention can be administered orally, in the form of aqueous suspensions in suitable mineral waters, for the treatment of poisoning and toxicosis caused by dietary imbalance, drug and alcohol abuse, and poisoning by pesticides or other environmental pollutants.

The catalytic properties of the zeolites of the invention also renew, stimulate and rebalance various enzymatic and metabolic activities which are crucial to the well-being of the body.

The zeolites of the invention can be formulated in forms suitable for oral administration, such as cachets, tablets, capsules or suspensions, possibly mixed with suitable vehicles or carriers. The administration of approx. 5 g a day of the zeolites of the invention is indicated in most cases, although the dose can obviously be varied as required. The treatment can continue for several months in view of the total absence of side effects.

The example below illustrates the invention in greater detail.

### EXAMPLE

In order to be conveniently used, the zeolites must be pre-activated by suitable procedures constituted by treatment with saline solutions (NaCl) which enhance their special application characteristics (their cation exchange capacity increases in a neutral to basic environment). 50 l of hyperthermal water (temperatures of 40/60°C at acid or neutral springs), particularly rich in minerals with a high catalytic potential (selenium, cobalt, sulphur, manganese, magnesium, zinc, etc.), is therefore added to 100 kg of zeolite powder obtained directly from a suitable deposit so that these fractions are adsorbed by the zeolite and subsequently released after oral administration. After the ultra-micronisation and ultra-ionisation steps in a humid environment (highspeed turboemulsifiers), 10 kg of unrefined sea salt (pH=7) per 100 Kg of zeolite is added and ultra-micronisation is continued for a further 24 hours in SS containers with suitable crushing discs. The mineral suspension thus obtained is first filtered through suitable circular vibrating screens (purpose-made) and then subjected to high magnetic fields (initially also used in the solid state, before the procedures) which are essential to orient the ion arrangement of the atoms and remove the ferrous component (metal iron), which accounts for a total of 5% of the suspension and is harmful for application purposes (Fenton's reaction). The filtrate is then subjected to the action of special hydropneumatic presses to extract the residual liquid component, so that the pressed ore contains approx. 15% water. The pressed ore is then positioned in suitable containers inserted into electric ovens (40°C for 24 hours) with conditioned hyperventilation, where the aqueous component is reduced to 7%. The powders thus obtained are packaged in suitable vacuum packs to maintain their application properties intact.

## Claims

1. The use of a zeolite consisting of 60% chabazite + phillipsite, 18% sanidine, 15% biotite and 7% analcime for the preparation of oral compositions for the treatment of poisoning or toxicosis.

2. The use according to claim 1, for the preparation of oral compositions for the treatment of toxicosis caused by dietary imbalance, drug and alcohol abuse, poisoning by pesticides and environmental pollutants.

## Patentansprüche

1. Die Verwendung eines Zeolits bestehend aus 60% Chabazit + Phillipsit, 18% Sanidin, 15% Biotit und 7% Analcim zur Herstellung von oralen Zusammensetzungen für die Behandlung von Vergiftung oder Toxikose.

2. Die Verwendung gemäß Anspruch 1 zur Herstellung von oralen Zusammensetzungen für die Behandlung von Toxikose, die durch unausgewogene Ernährung, Drogen- und Alkoholmissbrauch, Vergiftung durch Pestizide oder andere umwcltverschmutzende Stoffe verursacht wird.

## Revendications

1. L'utilisation d'une zéolite constituée de 60% de chabazite + phillipsite, 18% sanidine, 15% biotite et de 7% analcime pour la préparation de compositions orales pour le traitement de l'empoisonnement ou de la toxicose.

2. Utilisation selon la revendication 1, pour la préparation de compositions orales pour le traitement de la toxicose causée par un déséquilibre alimentaire, la toxicomanie et l'alcoolisme, l'empoisonnement par les pesticides et les polluants de l'environnement.
